# EUROPEAN PATENT APPLICATION

(11) **EP 0 574 844 A1**
(43) Date of publication of application: **22.12.1993**
(21) Application number: 93109376.9
(22) Date of filing: 11.06.1993
(51) Int. Cl.: C12Q 1/00, C12Q 1/32, C12Q 1/40

(54) **Method for the automated determination of isoenzymatic profiles and relevant equipment**

(30) Priority: 15.06.1992 IT FI920126
(71) Applicant: SCLAVO DIAGNOSTICI S.r.l., I-53100 Siena (IT)
(72) Inventor: Berti, Duccio, I-53034 Colle Val d'Elsa (Siena) (IT); Berardi, Anna, I-53100 Siena (IT); Bardelli, Fabrizio, I-53100 Siena (IT)
(74) Representative: Gervasi, Gemma, Dr.

(57) **Abstract**

Method for the automated determination of isoenzymatic profiles allowing a quick and highly sensitive data collection and processing through clustering techniques, and relevant equipment, are described.

## Description

### Field of the invention

The present invention refers to a method for the automated determination of isoenzymatic profiles, according to which the samples to be analyzed are treated with reagents suitable for producing a variance of response of the activity of the different isoenzymes. Measured variances are computer-processed according to the clustering techniques and compared with a reference standard obtained by acting likewise on the corresponding pure enzymes.

### State of the art

The analysis of the isoenzymatic components of a particular enzymatic activity is still scarcely exploited. The reason for the infrequent use of said analysis depends on the need to employ very expensive immunochemical reagents, on the laboriousness of the electrophoretic techniques to be applied, mainly on the lack of a complete fully automated pattern of diagnostically significant isoenzymes to be followed. The possibility of benefitting from a new method securing a quick and sensitive identification of the isoenzymatic components of a diagnostic sample appears to be a clearcut advantage.

### Detailed description of the invention

The present invention allows solving the aforesaid problems thanks to a method exploiting the different behaviour of each isoenzyme when compared to the others as an effect of an even slight change in some chemo-physical parameters of the reagents used for the determination of said isoenzymes. Moreover the invetion refers also to the equipment allowing the measurement and suitable processing of such variations.

It ought to be considered that changes in isoenzymatic activity ensuing a variation in chemo-physical parameters are often minor ones and such as to be practically useless for diagnostic purposes, if taken individually. It is, therefore, necessary to measure such changes globally, to distribute the collected data in a multi-dimensional space, and then apply suitable cluster analysis methods [see, for instance, Vogt W., Nagel D. "Cluster Analysis in Diagnosis", Clin. Chem., 38/2: 182-198 (1992)].

The equipment for the application of the method under the invention consists in a multi-purpose instrument, where the samples to be examined are accommodated and then treated with reagents suitable for modifying the activity of the various isoenzymes. Such changes are measured by means of detecting systems and the data thus obtained are processed by a computerized system into a data matrix, whose columns correspond to the response brought about by a particular reagent and whose rows represent the variance of the response of a sample vs. varying methods. Matrix data will be obviously normalized so as to nullify variances due to differing isoenzyme concentrations in tested samples, otherwise said concentration differences would result to be the main cause of measured variances, hence would conceal the desired information. All matrix data will also be normalized so that their absolute magnitude be reduced as next as possible to the unit. Then, all collected data will be processed according to a multivaried approach known as clustering technique [see Barrai I. "Introduzione all'analisi multivariata" (Introduction to Multivaried Analysis), Edagricole, Bologna (1986)]. The described processing of various pure enzymes allows plotting a reference standard for storing.

As is obvious, reference standards may be constructed on the sole basis of isoenzymes whose presence or absence has a diagnostic meaning, not of all isoenzymes of a given kind. Not only do such reduced models fail to lessen the method reliability, they are, instead, capable of enhancing the capabilities of the method described herein.

Test serum samples containing various isoenzymes undergo a similar treatment. From a comparison of the obtained data vs. the reference standard, the presence of looked-for isoenzymes can be determined easily and accurately. Due stress should be given to the fact that method repetition on different standards always gives the same pattern.

Among the reagents capable of causing a variance in isoenzymatic activity, it is possible to include acids or bases (varying the solution pH), saline solutions (varying the ionic force), activators or inhibitors of isoenzymatic activity etc. The method which the present invention refers to is now going to be better described with an example conveyed by way of clarification, not of limitation.

### EXAMPLE

Serum test samples were used for the determination of the presence of LDH and amylase isoenzymes.

The basic reagent for the measurement of the LDH enzymatic activity consisted of:

| | |
|---|---|
| Phosphate buffer | 50 mmoles/l pH 7.5 |
| Sodium pyruvate | 0.6 mmoles/l |
| Sodium azide | 1.0 g/l |
| NADH | 0.18 mmoles/l |

The basic reagent for the measurement of amylase activity consisted of:

| | |
|---|---|
| PIPES buffer | 70 mmoles/l pH 7.5 |
| p-NPG7 | 1.5 mmoles/l |
| Glucoamylase | 10 IU/l |
| Maltase | 10 IU/l |
| KCl | 50 mmoles/l |
| CaCl₂ | 5 mmoles/l |

Serum samples were added with basic reagent whose pH had been modified by addition of NaOH 0.1 N at intervals of 0.5 units (i.e. raised respectively to 8.0, 8.5 and 9.0). Four variances of isoenzymatic activity were thus measured by means of the Mitsubishi multipurpose instrument Z 818.

Two further series of measurements were made by varying the basic reagent ionic force with NaCl, respectively added at a concentration of 50 and 100 mmoles/l.

Test samples consisted of solutions containing the five LDH pure isoenzymes - obtained from human placenta according to the known methods -, human pancreatic amylase - supplied by Calbiochem Biochemical/Immunochemical (product No. 17153) -, and human saliva amylase - supplied by Sigma (product No. 0521).

Isoenzymatic activity variances were measured as follows: on samples containing LDH, by applying the six aforesaid conditions (4 pH variations plus 2 ionic force variations); on samples containing amylase, by applying the 4 pH variations only.

Determinations were conducted by means of the Mitsubishi multipurpose instrument Z 818; data were processed by means of IBM Personal System/2 model 60 using a main component projection programme with subsequent clustering [see above literature], suitably adjusted when required.

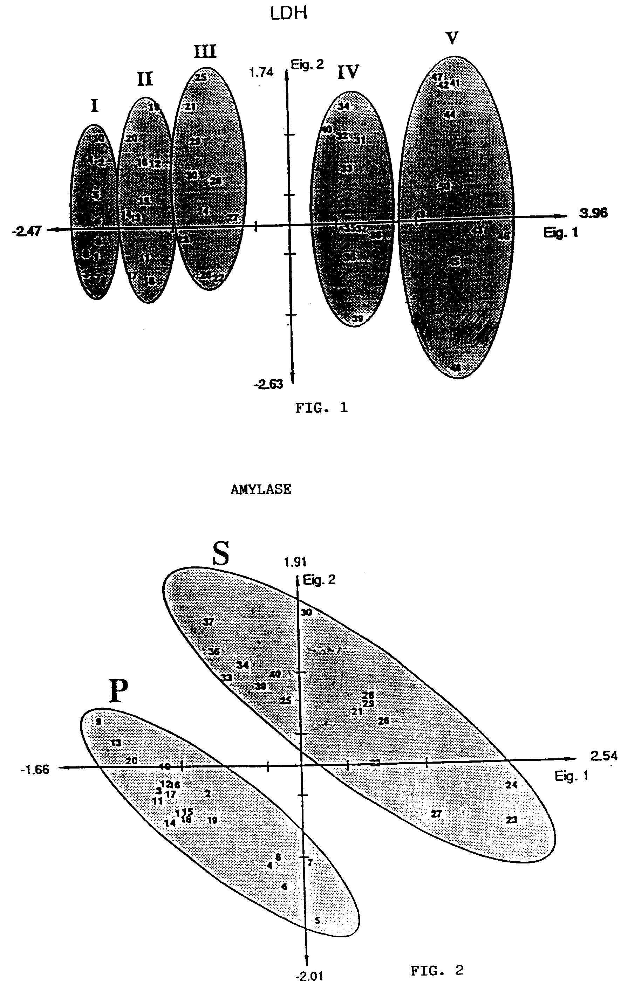

Figures 1 and 2 show the visualization of the clusters of pure isoenzymes of LDH and amylase, respectively salivary (S) and pancreatic (P), on an autovectorial plane; indices on axes are pure numbers expressing Euclidean distances.

After defining reference standards for pure LDH and amylase isoenzymes, as aforesaid, actual serum samples were analyzed.

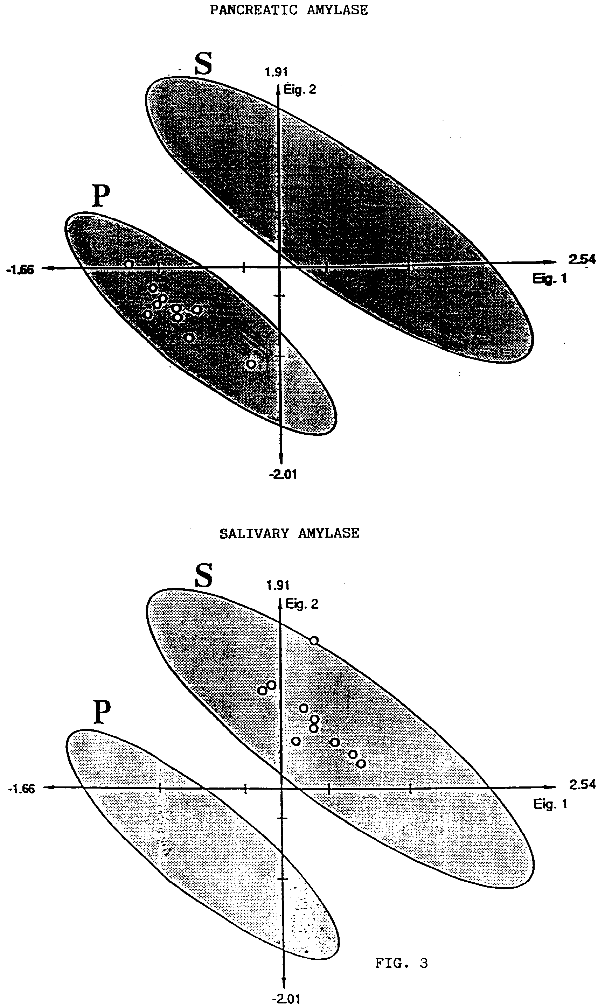

A set of 20 serum samples (10 containing pancreatic amylase and 10 containing salivary amylase), with high overall amylase activity, were analyzed according to the described technique. Results were as per Figure 3 (wherein the meaning of the values recorded on axes is as indicated above).

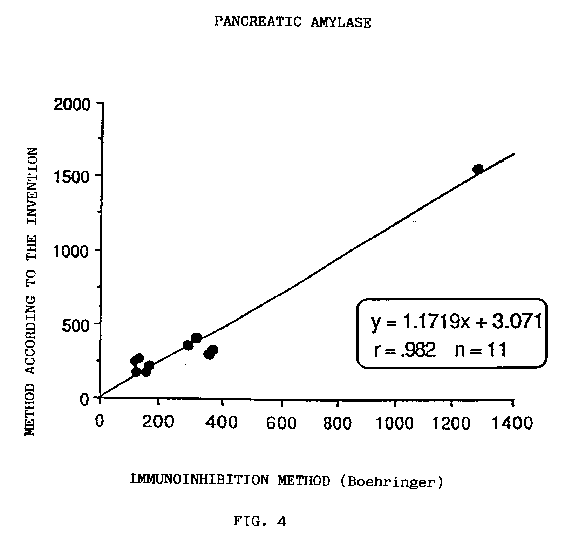

The 10 samples containing pancreatic amylase were analyzed also according to the standard immunoinhibition techniques (Boehringer Mannheim). Results were as per Figure 4.

In Figure 4, abscissas and ordinates show the quantities (expressed in IU/l) of pancreatic isoenzyme, respectively measured by the technique described herein and the traditional immunoinhibition method.

As may be seen, the slope of the straight line expressing the ratio between the values given by the two methods was practically equal to 1 (1.1719); data correlation was also very satisfactory (r = 0.982).

As far as LDH is concerned, two samples were analyzed. One of them showed a clearly higher activity (900 IU/l at 37°C) due to the presence of isoenzyme 1 and, to a lower extent, of isoenzyme 2. This result suggests the presence of myocardial infarction.

The other sample showed a lower overall activity (500 IU/l at 37°C), to be correlated to an increase in hepatic isoenzymes 4 and 5.

## Claims

1. Method for the automated determination of isoenzymatic profiles, according to which the samples to be analyzed are treated with reagents suitable for producing a variance of response of the activity of the different isoenzymes, the measured variances are computer-processed according to clustering techniques and compared with a reference standard obtained by acting likewise on the corresponding pure enzymes.

2. The method according to claim 1, wherein the reagents used to bring about response variance are acids, bases, varying ionic force solutions, activators or inhibitors of isoenzymatic activity.

3. The method according to claim 2, wherein two different reagents at least are used and, consequently, two different variances at least are measured.

4. The method according to claim 3 for the determination of LDH and amylase isoenzymes.

5. Equipment for applying the method according to claim 1, consisting of a multipurpose apparatus and a computerized system using a main component projection programme with subsequent clustering.
